# EUROPEAN PATENT SPECIFICATION

(11) **EP 1 475 123 B1**
(45) Date of publication and mention of the grant of the patent: **10.12.2008**
(21) Application number: 04101999.3
(22) Date of filing: 07.05.2004
(51) Int. Cl.: A61M 25/02

(54) **Catheter stabilizing device**
Vorrichtung zum Stabilisieren eines Katheters
Dispositif pour la stabilisation d'un cathéter

(30) Priority: 09.05.2003 IT BO20030285
(43) Date of publication of application: 10.11.2004
(73) Proprietor: BELLCO S.p.A., 20121 Milano (IT)
(72) Inventor: Greco, Francesco, 22063 Cantu (IT)
(74) Representative: Jorio, Paolo

(56) References cited:
- WO-A-00/71016
- WO-A-03/062890
- US-A- 5 509 902
- US-B1- 6 342 120
- US-B1- 6 554 233

## Description

The present invention relates to a catheter stabilizing device.

More specifically, the present invention relates to a catheter stabilizing device of the type described, for example, in US Patent 6,342,120, and comprising a sleeve, which is slid onto a catheter and fixed axially along the catheter, for example, by means of adhesive or an ultrasonic weld.

Known stabilizing devices of the above type have various drawbacks, mainly due to the sleeve being fixed permanently in a given position along the catheter.

Another type of catheter stabilizing device is disclosed, for example, in International Patent Application WO-00/71016-A1 and comprises a flexible sleeve, which is slid onto a catheter, and is fixed axially along the catheter by means of a tubular body fitted about the sleeve to compress the sleeve crosswise to its longitudinal axis.

It is an object of the present invention to provide a catheter stabilizing device designed to eliminate the aforementioned drawbacks, and which is cheap and easy to produce.

According to the present invention, there is provided a catheter stabilizing device as claimed in the attached Claims.

A non-limiting embodiment of the present invention will be described by way of example with reference to the accompanying drawings, in which:

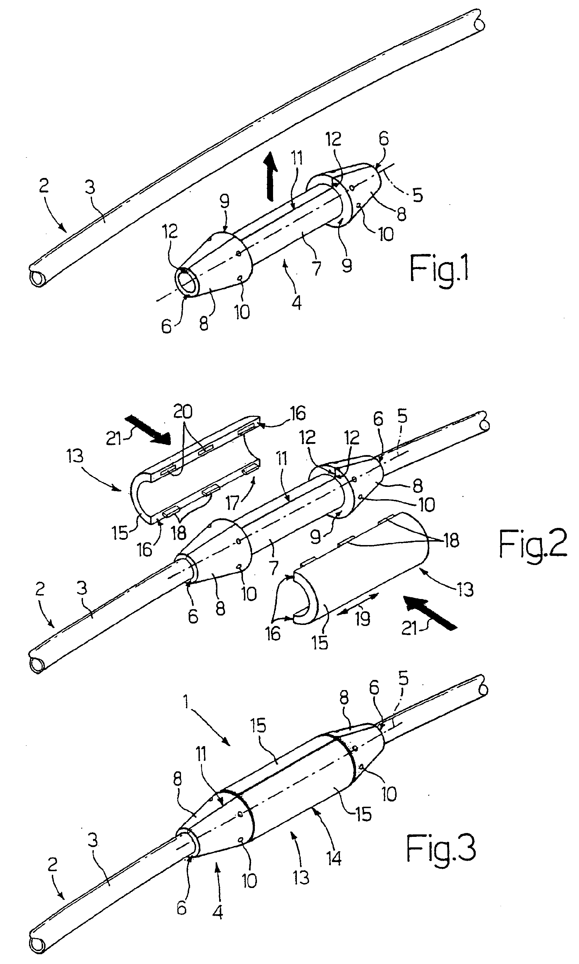

Figures 1 to 3 show views in perspective of a preferred embodiment of the stabilizing device according to the present invention at three stages in assembly to the relative catheter.

With reference to Figures 1 to 3, number 1 indicates as a whole a stabilizing device for fixing a catheter 2 inside a relative subcutaneous duct (not shown). In the example shown, catheter 2 comprises an elongated conduit 3 having a substantially circular cross section. Obviously, in alternative embodiments not shown, the number of conduits 3 is other than one, and the relative cross section is other than circular.

Device 1 comprises a tubular sleeve 4 made of flexible material, preferably, though not necessarily, silicone, and which has a longitudinal axis 5, a substantially circular cross section, and an inside diameter substantially equal to an outside diameter of conduit 3.

Sleeve 4 is bounded axially by two annular surfaces 6 perpendicular to axis 5, and comprises a substantially cylindrical central portion 7; and two substantially truncated-cone-shaped lateral portions 8 located at opposite ends of portion 7 and diverging towards portion 7 so as each to define a shoulder 9 perpendicular to axis 5 and facing shoulder 9 of the other portion 8.

Each portion 8 has a number of holes 10 spaced about axis 5 and for stabilizing device 1 inside said subcutaneous duct (not shown) by means of respective stitches.

Sleeve 4 is of a given length measured parallel to axis 5, and has a slit 11 extending between surfaces 6 and along the whole length of sleeve 4, and defining two facing flat longitudinal edges 12, which extend substantially parallel to axis 5 and are parted to fit sleeve 4 onto conduit 3.

Once fitted onto conduit 3, sleeve 4 is clamped axially along conduit 3 by means of a clamping device 13 comprising a cylindrical tubular body 14, which is fitted to portion 7 and between portions 8, is coaxial with axis 5, and has an inside diameter smaller than the outside diameter of portion 7, so as to compress sleeve 4 radially on conduit 3.

Tubular body 14 is of a length, measured parallel to axis 5, approximately equal to but no greater than the length of portion 7, also measured parallel to axis 5, and comprises two shells 15, each of which is made of rigid material, preferably, though not necessarily, a polymer, such as polyurethane, is semicylindrical, and is bounded by two flat longitudinal edges 16 extending parallel to each other and to axis 5, and which are positioned contacting edges 16 of the other shell 15.

The two shells 15 are fitted together by means of a fastening device 17, which, for each shell 15, comprises a number of (in the example shown, three) teeth 18 projecting from one of relative edges 16 and aligned with one another in a direction 19 parallel to axis 5; and a number of slots 20 equal in number to teeth 18 and formed in the other edge 16.

When shells 15 are moved with respect to each other in a direction 21 crosswise to axis 5 and to direction 19, each slot 20 of each shell 15 receives a respective tooth 18 on the other shell 15, and teeth 18 are locked inside respective slots 20 by means of a known click-in mechanism.

## Claims

1. A catheter stabilizing device, the device comprising a flexible sleeve (4) having a given longitudinal axis (5) and a given length measured parallel to said axis (5) and being fitted about at least one catheter (2); and clamping means (13) for clamping the sleeve (4) along the catheter (2), said clamping means (13) comprising a tubular body (14) fitted about said sleeve (4) to compress the sleeve (4) crosswise to said axis (5); **characterized in that** the sleeve (4) has a slit (11), which extends the whole of said length, and defines two edges (12) which are parted when fitting the sleeve (4) onto the catheter (2), and **in that** the sleeve (4) comprises a central portion (7) of constant cross section and two lateral portions (8) located at opposite ends of said central portion (7) and diverging towards the central portion (7); the tubular body (14) being fitted to said central portion (7) and between said lateral portions (8).

2. A device as claimed in Claim 1, wherein said tubular body (14) comprises two shells (15), and fastening means (17) for fastening the two shells (15) to each other.

3. A device as claimed in Claim 2, wherein each shell (15) has two further edges (16) positioned substantially contacting the further edges (16) of the other shell (15); said fastening means (17) comprising, for each said shell (15), a number of teeth (18) projecting from one of the relative further edges (16), and a number of slots (20) formed in the other relative further edge (16); and each slot (20) being shaped and located to receive a respective said tooth (18) when said shells (15) are moved with respect to each other in a direction (21) crosswise to said axis (5).

4. A device as claimed in any one of the foregoing Claims, wherein the tubular body (14) is of a length, measured parallel to said axis (5), approximately equal to but no greater than the length of said central portion (7) also measured parallel to said axis (5).

5. A device as claimed in any one of the foregoing Claims, wherein the catheter (2), the sleeve (4), and the tubular body (14) have respective substantially circular cross sections; the sleeve (4) having an inside diameter substantially equal to an outside diameter of the catheter (2), and an outside diameter smaller than an inside diameter of the tubular body (14).

6. A catheter assembly comprising at least one catheter (2), and a stabilizing device as claimed in any of Claims 1 to 5.

## Patentansprüche

1. Kathederstabilisierungsvorrichtung, wobei die Vorrichtung eine um mindestens einen Katheder (2) angebrachte flexible Hülse (4) mit einer festgelegten Längsachse (5) und einer parallel zu dieser Achse gemessenen festgelegten Länge aufweist; und eine Klemmeinrichtung (13) zum Klemmen der Hülse (4) entlang des Katheders (2), wobei die Klemmeinrichtung (13) einen röhrenförmigen Körper (14) umfasst, der um die Hülse (4) angebracht ist, um die Hülse (4) quer zu der Achse (5) zusammenzudrücken; **dadurch gekennzeichnet, dass** die Hülse (4) einen Schlitz (11) aufweist, der sich über die gesamte Länge erstreckt und zwei Flanken (12) definiert, die voneinander getrennt werden, wenn die Hülse (4) auf den Katheder (2) angebracht wird, und dadurch, dass die Hülse (4) einen mittleren Abschnitt (7) mit einem konstanten Querschnitt und zwei seitliche Abschnitte (8) aufweist, die an einander entgegengesetzten Enden des mittleren Abschnitts (7) angeordnet sind und hin zu dem mittleren Abschnitt (7) auseinanderlaufen; wobei der röhrenförmige Körper (14) an dem mittleren Abschnitt (7) und zwischen den seitlichen Abschnitten (8) angebracht ist.

2. Vorrichtung nach Anspruch 1, wobei der röhrenförmige Körper (14) zwei Hüllenteile (15) und eine Befestigungseinrichtung (17) umfasst, um die zwei Hüllenteile (15) aneinander zu befestigen.

3. Vorrichtung nach Anspruch 2, wobei jedes Hüllenteil (15) zwei weitere Flanken (16) aufweist, welche so positioniert sind, dass sie im Wesentlichen mit den weiteren Flanken (16) des anderen Hüllenteils in Kontakt stehen; wobei die Befestigungseinrichtung (17) für jedes der Hüllenteile (15) eine Anzahl von Zähnen (18) aufweist, die aus einer der jeweiligen weiteren Flanken (16) hervorstehen, und eine Anzahl von Schlitzen (20), die in der anderen jeweiligen weiteren Flanke (16) gebildet sind; und wobei jeder Schlitz (20) so geformt und angeordnet ist, dass er dazu fähig ist, einen zugehörigen Zahn (18) aufzunehmen, wenn die Hüllenteile (15) gegeneinander in einer Richtung (21) quer zu der Achse (5) bewegt werden.

4. Vorrichtung nach einem der vorhergehenden Ansprüche, wobei der rohrförmige Körper (14) parallel zu der Achse (5) gemessen eine Länge aufweist, die ungefähr gleich der, aber nicht größer als die ebenfalls parallel zu der Achse (5) gemessenen Länge des mittleren Abschnitts (7) ist.

5. Vorrichtung nach einem der vorhergehenden Ansprüche, wobei der Katheder (2), die Hülse (4) und der röhrenförmige Körper (14) jeweils im Wesentlichen kreisförmige Querschnitte aufweisen; die Hülse (4) einen Innendurchmesser aufweist, der im Wesentlichen gleich einem Außendurchmesser des Katheders (2) ist, und einen Außendurchmesser, der kleiner als ein Innendurchmesser des röhrenförmigen Körpers (14) ist.

6. Kathederbaugruppe, die mindestens einen Katheder (2) und eine Stabilisierungsvorrichtung nach einem der Ansprüche 1 bis 5 umfasst.

## Revendications

1. Dispositif de stabilisation de cathéter, le dispositif comprenant un manchon flexible (4) ayant un axe longitudinal donné (5) et une longueur donnée mesurée parallèlement audit axe (5) et ajusté autour d'au moins un cathéter (2) ; et un moyen de serrage (13) pour serrer le manchon (4) le long du cathéter (2), ledit moyen de serrage (13) comprenant un corps tubulaire (14) ajusté autour dudit manchon (4) pour comprimer le manchon (4) transversalement audit axe (5) ; **caractérisé en ce que** le manchon (41) a une fente (11), qui s'étend sur la totalité de ladite longueur et définit deux bords (12) qui sont divisés lors de l'ajustement du manchon (4) sur le cathéter (2), et **en ce que** le manchon (4) comprend une partie centrale (7) de section transversale constante et deux parties latérales (8) placées aux extrémités opposées de ladite partie centrale (7) et divergeant vers la partie centrale (7) ; le corps tubulaire (14) étant ajusté sur ladite partie centrale (7) et entre lesdites parties latérales (8).

2. Dispositif selon la revendication 1, dans lequel ledit corps tubulaire (14) comprend deux coques (15) et un moyen de fixation (17) pour fixer les deux coques (15) l'une à l'autre.

3. Dispositif selon la revendication 2, dans lequel chaque coque (15) a deux autres bords (16) positionnés sensiblement en contact avec les autres bords (16) de l'autre coque (15), ledit moyen de fixation (17) comprenant, pour chaque dite coque (15), un certain nombre de dents (18) faisant saillie de l'un des autres bords concernés (16), et un certain nombre de fentes (30) formées dans l'autre bord concerné (16) ; et chaque fente (20) étant façonnée et située pour recevoir une dite dent respective (18) lorsque lesdites coques (15) sont déplacées l'une par rapport à l'autre dans une direction (21) transversale audit axe (5).

4. Dispositif selon l'une quelconque des revendications précédentes, dans lequel le corps tubulaire (14) a une longueur, mesurée parallèlement audit axe (5), à peu près égale, mais pas supérieure à la longueur de ladite partie centrale (7) également mesurée parallèlement audit axe (5).

5. Dispositif selon l'une quelconque des revendications précédentes, dans lequel le cathéter (2), le manchon (4) et le corps tubulaire (14) ont des sections transversales respectives sensiblement circulaires ; le manchon (4) ayant un diamètre interne sensiblement égal à un diamètre externe du cathéter (2), et un diamètre externe plus petit qu'un diamètre interne du corps tubulaire (14).

6. Assemblage de cathéter comprenant au moins un cathéter (2) et un dispositif de stabilisation selon l'une quelconque des revendications 1 à 5.
